(19) Europäisches Patentamt
European Patent Office
Office européen des brevets

(11) Veröffentlichungsnummer: **0 207 000**
B1

(12) # EUROPÄISCHE PATENTSCHRIFT

(45) Veröffentlichungstag der Patentschrift: **08.11.89**

(51) Int. Cl.[4]: **C 07 D 241/20,** C 07 D 405/12, C 07 D 407/12, A 01 N 43/60

(21) Anmeldenummer: **86810274.0**

(22) Anmeldetag **16.06.86**

(54) **Mikrobizide.**

(30) Priorität: **21.06.85 CH 2650/85**

(43) Veröffentlichungstag der Anmeldung: **30.12.86 Patentblatt 86/52**

(45) Bekanntmachung des Hinweises auf die Patenterteilung: **08.11.89 Patentblatt 89/45**

(84) Benannte Vertragsstaaten: **AT BE CH DE FR GB IT LI LU NL SE**

(56) Entgegenhaltungen:
**EP-A- 0 151 062**
**GB-A- 2 124 615**
**US-A- 4 359 576**

(73) Patentinhaber: **CIBA-GEIGY AG, Klybeckstrasse 141, CH-4002 Basel (CH)**

(72) Erfinder: **Eckhardt, Wolfgang, Dr., Breslauerstrasse 14, D-7850 Lörrach (DE)**
Erfinder: **Zondler, Helmut, Dr., Oberwilerstrasse 49, CH-4103 Bottmingen (CH)**
Erfinder: **Nyfeler, Robert, Dr., Bärenfelserstrasse 8, CH-4057 Basel (CH)**

**EP 0 207 000 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue (Arylmethylimino)-pyrazine der nachstehenden Formel I. Die Erfindung betrifft ferner die Herstellung dieser Substanzen sowie Mittel, die als Wirkstoff mindestens eine dieser Verbindungen enthalten. Sie betrifft die Herstellung der Mittel sowie die Verwendung der Wirkstoffe oder der Mittel zur Bekämpfung schädlicher Mikroorganismen, vorzugsweise pflanzenschädigender Pilze und Bakterien. Die Erfindung betrifft überdies wichtige Zwischenprodukte der nachfolgend definierten Formeln II und VI.

Aus der US-Patentschrift US 4 359 576 ist bekannt geworden, dass bestimmte N-Pyrazinyl-N--benzylcarbamate fungizid und pflanzenwuchsregulatorisch wirksam sind.

Demgegenüber wurde nun gefunden, dass bestimmte (Arylmethylimino)-pyrazine ebenfalls fungizid wirksam sind.

Bei den erfindungsgemässen Verbindungen handelt es sich um solche der allgemeinen Formel I

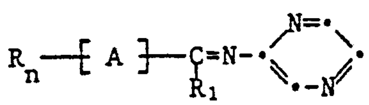

(I)

worin

A für Phenyl, Naphthyl, Biphenyl, Phenoxyphenyl oder Phenylthiophenyl steht;

R Halogen, Cyano, Nitro, $C_1$-$C_4$-Alkyl, $C_1$-$C_4$-Alkoxy, $C_1$-$C_4$-Haloalkoxy, $C_1$-$C_4$-Haloalkyl oder Di($C_1$-$C_4$-alkyl)amino bedeutet;

n für 0, 1, 2, 3, 4 oder 5 steht;

$R_1$ eine der Gruppen $OR_2$, $SR_2$ oder $N(R_3)(R_4)$ repräsentiert, wobei

$R_2$ für $C_1$-$C_8$-Alkyl, $C_1$-$C_4$-Haloalkyl, $C_3$-$C_8$-Alkenyl, $C_3$-$C_8$-Alkinyl oder für einen unsubstituierten oder ein- bis dreifach durch R substituierten Rest aus der Reihe Phenyl, Benzyl, $C_3$-$C_7$-Cycloalkyl oder Furfuryl steht; und

$R_3$ und $R_4$ unabhängig voneinander für Wasserstoff oder $C_1$-$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder als ein weiteres Heteroatom N, O oder S enthält.

Unter dem Begriff Alkyl selbst oder als Bestandteil eines anderen Substituenten wie Alkoxy, Haloalkyl, Haloalkoxy etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl, Heptyl, Octyl usw. sowie ihre Isomeren, wie z.B. Isopropyl, Isobutyl, tert.-Butyl, Isopentyl usw. Die Vorsilbe Halo in der Bezeichnung eines Substituenten bedeutet hier und im folgenden, dass dieser Substituent einfach bis perhalogeniert auftreten kann. Halogen und Halo stehen stellvertretend für Fluor, Chlor, Brom oder Jod. Haloalkyl steht somit für einen einfach bis perhalogenierten Alkylrest, wie z.B. $CHCl_2$, $CH_2F$, $CCl_3$, $CH_2Cl$, $CHF_2$, $CH_2CH_2Br$, $C_2Cl_5$, $CH_2Br$, $CHBrCl$, $CH_2CF_3$ usw., vorzugsweise für $CF_3$. Alkenyl steht z.B. für Propenyl-(1), Allyl, Butenyl-(1), Butenyl-(2) oder Butenyl-(3) sowie Ketten mit mehreren Doppelbindungen. Cycloalkyl steht je nach Zahl der genannten Kohlenstoffatome z.B. für Cyclopropyl, Cyclobutyl, Cyclopentyl, Cyclohexyl, Cycloheptyl, Cyclooctyl usw. Alkinyl bedeutet z.B. Propinyl-(2), Propargyl, Butinyl-(1), Butinyl-(2) usw. vorzugsweise Propargyl.

Folgende Wirkstoffgruppen sind auf Grund ihrer ausgeprägten mikrobiziden, insbesondere pflanzenfungiziden Aktivität bevorzugt:

Gruppe Ia: Verbindungen der Formel I, worin A für Phenyl steht; R Halogen, Cyano, Methyl, Ethyl, Methoxy, Ethoxy, $CF_3$, $C_1$-$C_2$-Haloalkoxy, $N(CH_3)_2$ oder $N(C_2H_5)_2$ bedeutet; n für 1, 2 oder 3 steht; $R_1$ eine der Gruppen $OR_2$ oder $SR_2$ repräsentiert; wobei $R_2$ für $C_1$-$C_8$-Alkyl, $C_3$-$C_8$-Alkenyl, $C_3$-$C_8$-Alkinyl oder für einen unsubstituierten oder ein- bis dreifach durch Halogen, Cyano, Methyl, Ethyl, Methoxy, Ethoxy, $CF_3$, $C_1$-$C_2$-Haloalkoxy oder $N(C_1$-$C_2$-Alkyl$)_2$ substituierten Rest aus der Reihe Phenyl, Benzyl, $C_5$-$C_6$-Cycloalkyl und Furfuryl steht.

Gruppe Ib: Verbindungen der Formel I, worin A für Phenyl steht; R Fluor, Chlor, Brom, Methyl, Methoxy oder $CF_3$ bedeutet; n für 1 oder 2 steht; $R_1$ für $OR_2$ oder $SR_2$ steht; wobei $R_2$ für $C_1$-$C_5$-Alkyl, $C_3$-$C_4$-Alkenyl, $C_3$-$C_4$-Alkinyl, Cyclopentyl, Cyclohexyl, Furfuryl oder einen unsubstituierten oder ein- bis dreifach durch Fluor, Chlor, Brom, Methyl, Methoxy oder $CF_3$ substituierten Rest aus der Reihe Phenyl und Benzyl bedeutet.

Gruppe Ic: Verbindungen innerhalb der Gruppe Ib, worin R für Fluor oder Chlor steht; n die 2- und 4-Position des Phenylrings repräsentiert; $R_1$ für $OR_2$ oder $SR_2$ steht; wobei $R_2$ für $C_1$-$C_5$-Alkyl, Allyl, Propargyl, Cyclohexyl, Benzyl oder unsubstituiertes oder ein-bis dreifach durch Fluor, Chlor, Brom, Methyl, Methoxy oder $CF_3$ substituiertes Phenyl bedeutet.

Gruppe Id: Verbindungen der Formel I, worin A für Phenoxyphenyl steht; n für 0, 1, 2, 3, 4 oder 5 steht; und die übrigen Substituenten wie in der Gruppe Ia definiert sind.

Gruppe Ie: Verbindungen der Formel I innerhalb der Gruppe Id, worin A für (p-Phenoxy)phenyl steht.

Gruppe If: Verbindungen der Formel I innerhalb der Gruppe Ie, worin R, $R_1$ und $R_2$ wie in Gruppe Ib definiert sind und n für 0, 1, 2, 3 oder 4 steht.

Gruppe Ig: Verbindungen der Formel I innerhalb der Gruppe If, worin, $R_1$ und $R_2$ wie in Gruppe Ic definiert sind, n für 0, 1, 2 oder 3 steht und R Fluor, Chlor, Methyl, Methoxy oder $CF_3$ bedeutet.

Besonders bevorzugte Einzelsubstanzen sind beispielsweise:

[2,4-Dichlorbenzyl-C-(tert.butylthio)imino]pyrazin (Nr. 1.7);

[2,4-Dichlorbenzyl-C-(isopropylthio)imino]pyrazin (Nr. 1.4).

Die Verbindungen der Formel I werden erfindungsgemäss dadurch hergestellt, dass man eine Verbindung der Formel II

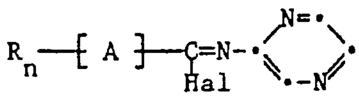

(II)

in Gegenwart einer Base mit einer Verbindung der Formel III

$R_1$-H (III)

umsetzt; dabei haben $R_n$, A und $R_1$ in den Formeln II und III die unter Formel I angegebenen Bedeutungen, Hal steht für Halogen, vorzugsweise für Chlor oder Brom.

Die Umsetzung von (II) mit (III) zu (I) erfolgt unter Halogenwasserstoffabspaltung. Günstige Reaktionstemperaturen liegen zwischen 0°C und +180°C, vorzugsweise +20° und +150°C bzw. am Siedepunkt des Lösungsmittels bzw. Lösungsmittelgemisches. Im allgemeinen ist die Verwendung von säurebindenden Mitteln bzw. Kondensationsmitteln (Basen) vorteilhaft. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Alkoholate wie z.B. Kaliumtert.-Butylat, Oxide und Hydroxide, Carbonate und Hydrogencarbonate von Alkali- und Erdalkalimetallen sowie die Salze der Essigsäure wie z.B. die Alkaliacetate.

Die Reaktion kann in Anwesenheit von reaktionsinerten Lösungs-oder Verdünnungsmitteln durchgeführt werden. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorethylen; Ether und etherartige Verbindungen wie Dialkylether (Diethylether, Diisopropylether, tert.-Butylmethylether usw.), Anisol, Dioxan, Tetrahydrofuran; cyclische Sulfone wie Sulfolan; Nitrile wie Acetonitril, Proprionitril; N,N-dialkylierte Amide wie Dimethylformamid; Dimethylsulfoxid; Ketone wie Aceton, Diethylketon, Methylethylketon, N-Methylpyrrolidon und Gemische solcher Lösungsmittel untereinander.

Im übrigen können bei der Herstellung aller hierin genannten Ausgangs-, Zwischen- und Endprodukte grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Die Verbindungen der Formel III sind allgemeinen bekannt oder lassen sich nach an sich bekannten Methoden herstellen.

Die Verbindungen der Formel II sind neu. Aufgrund ihrer Struktur sind sie geradezu prädestiniert für die Herstellung der mikrobizid, aktiven Endprodukte der Formel I oder anderer Pestizide. Sie sind somit wertvolle Zwischenprodukte und bilden einen wichtigen Bestandteil der vorliegenden Erfindung.

Die Herstellung von Verbindungen der Formel II kann z.B. folgendermassen erfolgen:

Ein Aminopyrazin der Formel IV

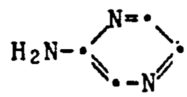

(IV)

wird vorzugsweise in Gegenwart einer Base mit einem Säurehalogenid, vorzugsweise dem Chlorid oder Bromid der Formel V

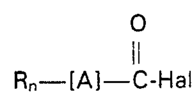

(V)

zu einer Verbindung der Formel VI

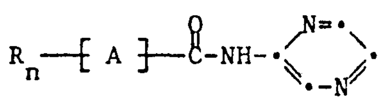

(VI)

N-acyliert und letzteres, auf an sich bekannte Weise (z.B. mit $SOCl_2$, $PBr_5$, $PCl_5$, N-Bromsuccinimid usw.) zu einer Verbindung der Formel II halogeniert, dabei haben die Substituenten $R_n$ und A in den Formeln V und VI die unter Formel I angegebenen Bedeutungen und Hal steht für Halogen, vorzugsweise Chlor oder Brom. Die Umsetzung von (IV) mit (V) zu (VI) erfolgt unter den für die Reaktion von (II) mit (III) zu (I) genannten Bedingungen.

Bei der Herstellung der Verbindungen der Formel I, ist es nicht zwingend notwendig, dass man die Verbindung der Formel II in ihrer isolierten Form einsetzt, vielmehr kann man auch die Verbindung der Formel VI vorlegen, diese durch Halogenierung (z.B. mit $SOCl_2$) in das Halogenid der Formel (II) (Chlorid) überführen und (II) in situ, erfindungsgemäss zu (I) umsetzen. Dieses Eintopfverfahren ist ebenfalls Bestandteil dieser Erfindung, da das Zwischenprodukt II hierbei intermediär auftritt und, falls gewünscht, aus dem Reaktionsmedium isoliert werden kann. Die Verbindungen der Formel VI sind somit unmittelbare Vorprodukte bei der Herstellung der Verbindungen der Formel I. Sie sind neu und überdies selbst mikrobizid wirksam. Sie zeigen u.a. sehr gute pflanzenfungizide Wirkung und sind z.B. auf dem gleichen Indikationsgebiet wie die Endprodukte der Formel I einsetzbar. Die Verbindungen der Formel VI sind ein Bestandteil vorliegender Erfindung.

Analog zur C = C-Doppelbindung führt die C = N-Doppelbindung in den Verbindungen der Formel I, aber auch in den Zwischenprodukten der Formel II, zu unterschiedlichen geometrischen Isomeren:

Verbindung der Formel I

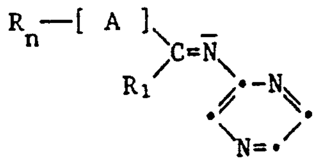

(I)

syn-$R_1$ oder anti-$R_n$—[A]—;

(trans-Form)

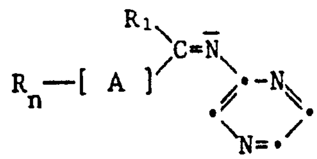

syn-$R_n$—[A]— oder anti-$R_1$

(cis-Form)

Verbindungen der Formel

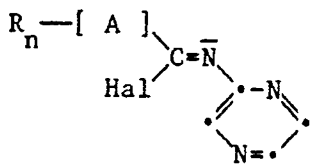

syn-Hal oder anti-$R_n$—[A]—;

(trans-Form)

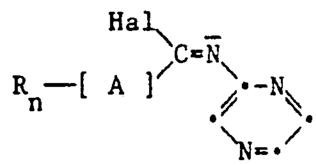

syn-$R_n$—[A]— oder anti-Hal

(cis-Form)

Im allgemeinen entsteht bei der Herstellung von Verbindungen der Formeln I und II ein Gemisch der cis- und trans-Form, wobei die thermodynamisch günstigere Form bevorzugt gebildet wird. Die Substanzen können ohne Auftrennung der Isomeren im Pflanzenschutz eingesetzt werden. Allerdings kann der Einsatz der reinen Isomeren zu einer Wirkungserhöhung führen.

Die Erfindung betrifft somit sämtliche isomeren Verbindungen der Formel I in reiner Form oder in beliebigem Zahlenverhältnis zueinander.

Das Herstellungsverfahren von Verbindungen der Formel I ist in seinen beschriebenen Varianten ein Bestandteil dieser Erfindung.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helmintosporium, Fusarium, Septoria, Cercospora und Alternaria). Überdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnen Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gehörnte, adsorptive Granulatträger kommen poröse Typen wie z.B. Bimsstein, Ziegelbruch, Sepiolit oder Bentonit, als nicht sorptive Trägermaterialien z.B. Calcit, oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylchlorin-Mischungen. Synthetische Phospholipide sind z.B. Dioctanoylphosphatidylchlolin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierbei gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkyrest der Alkylphenole enthalten können.

Weitere geeignete nichtionische Tenside sind die wasserlöslichen, 20 bis 250 Äthylenglykoläthergruppen und 10 bis 100 Propylenglykolethergruppen enthaltenden Polyethylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüber hinaus werden folgende Symbole verwendet: h = Stunde; d = Tag; min = Minute; RT = Raumtemperatur; N = Normalität; abs = absolut, wasserfrei, DMSO = Dimethylsulfoxid; DMF = Dimethylformamid. Druckangaben erfolgen in Millibar mb, oder Bar b. $C_6H_5$ charakterisiert stets eine Phenylgruppe. Eine substituierte Phenylgruppe z.B. ortho-, para-Dichlorphenyl wird dann dementsprechend als $C_6H_3Cl_2(2,4)$ abgekürzt.

1. *Herstellungsbeispiele*

*Beispiel 1.1:*

*Herstellung von 2-[2,4-Dichlorbenzyl-C-(tert.butylthio)imino]pyrazin*

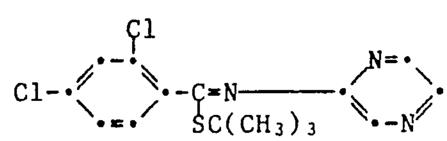

a) *Herstellung der Zwischenstufe N-(Pyrazinyl)--2,4-dichlorbenzimidchlorid*

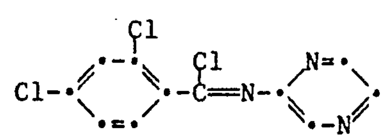

16,6 g (0,062 Mol) des entsprechenden Amides und 110 g (0,93 Mol) Thionylchlorid wurden über Nacht bei Siedetemperatur gerührt, wobei ein leichter Stickstoffstrom aufgesetzt wurde. Dann wurde das Thionylchlorid unter vermindertem Druck abgezogen und das zurückbleibende Öl zweimal mit Toluol versetzt und jedesmal gut eingeengt. Man erhält so praktisch quantitativ das Imidchlorid als braunes Öl.

b) *Herstellung der Titelverbindung*

8,9 g (0,031 Mol des hergestellten Imidchlorids und 3,6 g (0,04 Mol) tert-Butylmercaptan wurden

in 50 ml Tetrahydrofuran vorgelegt und bei 5°C mit 6,2 g (0,055 Mol) Kalium-tert-butylat in 50 ml Tetrahydrofuran tropfenweise umgesetzt. Dann wurde das Reaktionsgemisch 3 Std. bei Raumtemperatur ausgerührt und auf ca. 1 Liter schwach salzsaures Eiswasser gegossen und mit Essigsäureethylester 2 mal extrahiert. Die Essigesterlösung wurde 1 mal mit Wasser gewaschen, mit Natriumsulfat getrocknet, filtriert und eingeengt. Das Rohprodukt wurde über eine Kieselgel-Flach-Säule mit Essigester/Petroläther = 1/3 gereinigt. Man erhielt so 6,1 g Produkt.

Analog den beschriebenen Arbeitsweisen erhält man die nachfolgend genannten Verbindungen der Formel I:

TABELLE 1

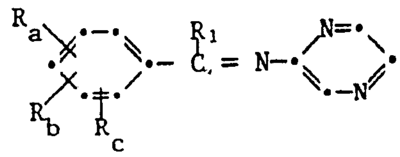

| Verb. Nr. | $R_a$ | $R_b$ | $R_c$ | $R_1$ | Physik. Konst. |
|---|---|---|---|---|---|
| 1.1 | 2-Cl | 4-Cl | H | $SCH_3$ | |
| 1.2 | 2-Cl | 4-Cl | H | $SCH_2H_5$ | |
| 1.3 | 2-Cl | 4-Cl | H | $SC_3H_7(n)$ | $n_D^{31}$ 1,6229 |
| 1.4 | 2-Cl | 4-Cl | H | $SC_3H_7(i)$ | Öl |
| 1.5 | 2-Cl | 4-Cl | H | $SC_4H_9(n)$ | |
| 1.6 | 2-Cl | 4-Cl | H | $SC_4H_9(s)$ | |
| 1.7 | 2-Cl | 4-Cl | H | $SC_4H_9(t)$ | Smp. 50-52°C |
| 1.8 | 2-Cl | 4-Cl | H | $SCH_2CH=CH_2$ | $n_D^{31}$ 1,6376 |
| 1.9 | 2-Cl | 4-Cl | H | $SCH_2C\equiv CH$ | |
| 1.10 | 2-Cl | 4-Cl | H | 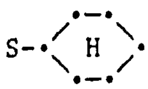 | |
| 1.11 | 2-Cl | 4-Cl | H | 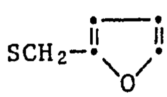 | $n_D^{50}$ 1,6431 |
| 1.12 | 2-Cl | 4-Cl | H | 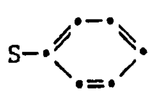 | |
| 1.13 | 2-Cl | 4-Cl | H | 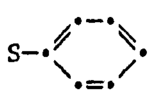 | Öl |
| 1.14 | 2-Cl | 4-Cl | H | 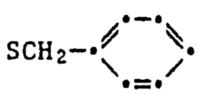 | $n_D^{50}$ 1,6532 |
| 1.15 | 2-Cl | 4-Cl | H | $SCH_2CH(CH_3)_2$ | |

TABELLE 1 (Fortsetzung)

| Verb. Nr. | $R_a$ | $R_b$ | $R_c$ | $R_1$ | Physik. Konst. |
|---|---|---|---|---|---|
| 1.16 | 2-Cl | 4-Cl | H | $OCH_3$ | |
| 1.17 | 2-Cl | 4-Cl | H | $OC_2H_5$ | |
| 1.18 | 2-Cl | 4-Cl | H | $OC_3H_7$(i) | $n_D^{50}$1,5655 |
| 1.19 | 2-Cl | 4-Cl | H | $OC_4H_9$(n) | |
| 1.20 | 2-Cl | 4-Cl | H | $OC_4H_9$(t) | $n_D^{50}$ 1,5578 |
| 1.21 | 2-Cl | 4-Cl | H | $OCH_2C{\equiv}CH$ | |
| 1.22 | 2-Cl | 4-Cl | H | 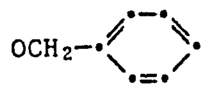 | |
| 1.23 | 2-Cl | 4-Cl | H | 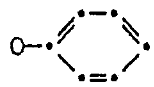 | |
| 1.24 | 2-Cl | 4-Cl | H | 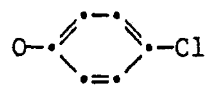 | Smp. 104-110°C |
| 1.25 | 2-Cl | 4-Cl | H | $NHC_2H_5$ | |
| 1.26 | 2-Cl | 4-Cl | H | $NHC_3H_7$(n) | |
| 1.27 | 2-Cl | 4-Cl | H | $NHC_4H_9$(n) | |
| 1.28 | 2-Cl | 4-Cl | H | $NH\text{-}\underset{}{CH}(CH_3)\text{-}CH_2CH_3$ | Smp. 147-149°C |
| 1.29 | 2-Cl | 4-Cl | H | 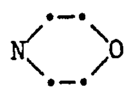 | |
| 1.30 | 2-Cl | 4-Cl | H | 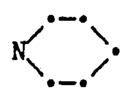 | |
| 1.31 | 2-Cl | 4-Cl | 6-Cl | $SC_3H_7$(i) | |
| 1.32 | 2-Cl | 4-Br | H | $SC_3H_7$(i) | |
| 1.33 | 2-cl | 4-Br | H | $SC_4H_9$(t) | |
| 1.34 | 2-Cl | 4-Br | H | $SCH_2CH=CH_2$ | |
| 1.35 | 2-Cl | 4-Br | H | 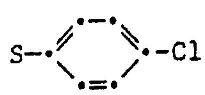 | |

TABELLE 1 (Fortsetzung)

| Verb. Nr. | $R_a$ | $R_b$ | $R_c$ | $R_1$ | Physik. Konst. |
|---|---|---|---|---|---|
| 1.36 | 2-Cl | 4-Br | H | 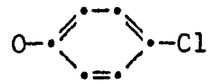 | |
| 1.37 | 2-Cl | 4-F | H | S-$C_4H_9$(t) | |
| 1.38 | 2-Cl | 4-F | H | $SC_3H_7$(i) | |
| 1.39 | 2-Cl | 4-F | H | 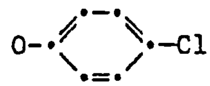 | |
| 1.40 | 2-Cl | 4-F | H | $CH_3$<br>NH-$CHCH_2CH_e$ | |
| 1.41 | 2-Cl | 4-Br | H | $CH_3$<br>NH-$CHCH_2CH_3$ | |
| 1.42 | H | 4-Cl | H | $SC_4H_9$(t) | |
| 1.43 | 2-$NO_2$ | 4-$NO_2$ | H | $SC_4H_9$(t) | |
| 1.44 | 2-$OCH_3$ | 4-$OCH_3$ | 6-$OCH_3$ | $SC_4H_9$(t) | |
| 1.45 | 3-Cl | 4-Cl | H | $SC_4H_9$(t) | |
| 1.46 | H | 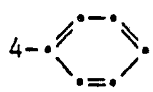 | H | $SC_4H_9$(t) | |
| 1.47 | H | 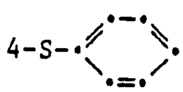 | H | $SC_4H_9$(t) | |
| 1.48 | 2-F | 4-Cl | H | $SC_3H_7$(i) | |
| 1.49 | 2-F | 4-Cl | H | $SC_4H_9$(t) | |
| 1.50 | 2-F | 4-Cl | H | 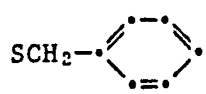 | |
| 1.51 | 2-F | 4-Cl | H | $SCH_2CH=CH_2$ | |
| 1.52 | 2-F | 4-Cl | H | 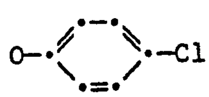 | |
| 1.53 | 2-F | 4-Cl | H | NH-$CHC_2H_5$<br>$CH_3$ | |

TABELLE 1 (Fortsetzung)

| Verb. Nr. | $R_a$ | $R_b$ | $R_c$ | $R_1$ | Physik. Konst. |
|---|---|---|---|---|---|
| 1.54 | 2-Br | 4-Br | H | $SC_4H_9(t)$ | |
| 1.55 | 2-Cl | 4-Cl | H | $OCH<^{CH_3}_{C_3H_7(n)}$ | |
| 1.56 | 2-Cl | 4-Cl | H | $OCH_2CF_3$ | |
| 1.57 | 2-F | 4-Cl | H | $OCH_2CF_3$ | |

TABELLE 2

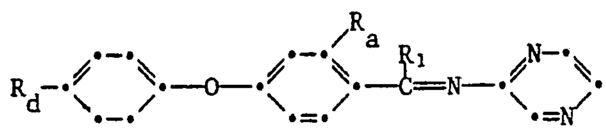

| Verb. Nr. | $R_a$ | $R_d$ | $R_1$ | Physik. Konst. |
|---|---|---|---|---|
| 2.1 | H | H | $SC_3H_7(i)$ | |
| 2.2 | H | H | $SC_4H_9(t)$ | |
| 2.3 | H | H | 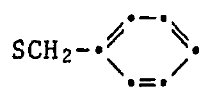 | |
| 2.4 | H | H | 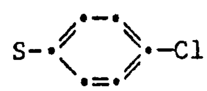 | |
| 2.5 | H | H | $OC_2H_5$ | |
| 2.6 | H | H | 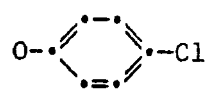 | |
| 2.7 | H | H | $SCH_2CH=CH_2$ | |
| 2.8 | H | H | $NHCHCH_2CH_3$<br>$CH_3$ | |
| 2.9 | $CH_3$ | Cl | $SC_3H_7(i)$ | |
| 2.10 | $CH_3$ | Cl | $SC_4H_9(t)$ | |
| 2.11 | $CH_3$ | Cl | 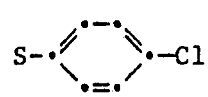 | |
| 2.12 | $CH_3$ | Cl | $SCH_2CH=CH_2$ | |

TABELLE 2 (Fortsetzung)

| Verb. Nr. | $R_a$ | $R_d$ | $R_1$ | Physik. Konst. |
|---|---|---|---|---|
| 2.13 | $CH_3$ | Cl | 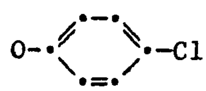 | |
| 2.14 | $CH_3$ | Cl | $NHCH(CH_3)CH_2CH_3$ | |
| 2.15 | Cl | Cl | $SC_3H_7(i)$ | |
| 2.16 | Cl | Cl | $SC_4H_9(t)$ | |
| 2.17 | Cl | Cl | 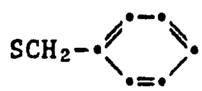 | |
| 2.18 | Cl | H | $SCH_2CH=CH_2$ | |
| 2.19 | Cl | H | $OC_2H_5$ | |
| 2.20 | Cl | H | 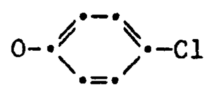 | |
| 2.21 | Cl | H | $NHCH(CH_3)CH_2CH_3$ | |
| 2.22 | Br | Br | $SC_4H_9(t)$ | |
| 2.23 | $CH_3$ | Br | $SC_4H_9(t)$ | |
| 2.24 | Cl | Br | $SC_4H_9(t)$ | |

2. *Formulierungsbeispiele für Wirkstoffe der Formel I*

(% = Gewichtsprozent)

| 2.1 *Emulsions-Konzentrate* | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykol-ether (36 Mol Ethylenoxid) | 5% | — | — |
| Tributylphenol-polyethylen-glykolether (30 Mol Ethylenoxid) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2.2 *Lösungen* | *a)* | *b)* | *c)* | *d)* |
|---|---|---|---|---|
| Wirkstoff aus den Tabellen | 80% | 10% | 5% | 95% |
| Ethylenglykol-monomethyl-ether | 20% | — | — | — |
| Polyethylenglykol MG 400 | — | 70% | — | — |
| N-Methyl-2--pyrrolidon | — | 20% | — | — |
| Epoxydiertes Kokosnussöl | — | — | 1% | 5% |
| Benzin (Siedegrenzen 160-190°C | — | — | 94% | — |

(MG = Molekulargewicht)

Die Lösungen sind zur Anwendung in Form kleinster Tropfen geeignet.

| 2.3 *Granulate* | a) | b) |
|---|---|---|
| Wirkstoff aus den Tabellen | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft.

| 2.4 *Stäubemittel* | a) | b) |
|---|---|---|
| Wirkstoff aus den Tabellen | 2% | 5% |
| Hochdisperse Kieselsäure | 1% | 5% |
| Talkum | 97% | — |
| Kaolin | — | 90% |

Durch inniges Vermischen auf Trägerstoffe mit dem Wirkstoff erhält man gebrauchsfertige Stäubemittel.

| 2.5 *Spritzpulver* | a) | b) | c) |
|---|---|---|---|
| Wirkstoff aus den Tabellen | 25% | 50% | 75% |
| Na-Ligninsulfonat | 5% | 5% | — |
| Na-Laurylsulfat | 3% | — | 5% |
| Na-Diisobutyl-naphthalinsulfonat | — | 6% | 10% |
| Octylphenolpolyethylen-glykolether (7-8 Mol Ethylenoxid) | — | 2% | — |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. man erhält Spritzpulver, die sich mit Wasser zu Suspensionen jeder gewünschten Konzentration verdünnen lassen.

| 2.6 *Emulsions-Konzentrat* | |
|---|---|
| Wirkstoff aus den Tabellen | 10% |
| Octylphenolpolyethylen-glykolether (4-5 Mol Ethylenoxid) | 5% |
| Ca-Dodecylbenzol-sulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 2.7 *Stäubemittel* | a) | b) |
|---|---|---|
| Wirkstoff aus den Tabellen | 5% | 8% |
| Talkum | 95% | — |
| Kaolin | — | 92% |

Man erhält anwendungsfertige Stäubemittel, indem der Wirkstoffe mit dem Träger vermischt und auf einer geeigneten Mühle vermahlen wird.

| 2.8 *Extruder-Granulat* | |
|---|---|
| Wirkstoff auf den Tabellen | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 *Umhüllungs-Granulat* | |
|---|---|
| Wirkstoff aus den Tabellen | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

3. *Biologische Beispiele:*

*Beispiel 3.1:*

*Wirkung gegen Puccinia graminis auf Weizen*

a) *Residual-protektive Wirkung*

Weizenpflanzen wurden 6 Tage nach der Aussaat mit einer aus Spritzpulver des Wirkstoffes hergestellten Spritzbrühe (0,006% Aktivsubstanz) besprüht. Nach 24 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) *Systemische Wirkung*

Zu Weizenpflanzen wurde 5 Tage nach der Aussaat eine aus Spritzpulver des Wirkstoffs hergestellte Spritzbrühe gegossen (0,002% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion. Verbindungen aus den Tabellen zeigten gegen Puccinia-Pilze eine gute Wirkung. Unbehandelte aber infizierte Kontrollpflanzen zeigten einen Puccinia-Befall von 100%. Die Verbindungen Nr. 1.4, 1.7 und 1.18 hemmten den Puccinia-Befall auf 0 bis 5%.

*Beispiel 3.2:*

*Wirkung gegen Cercospora arachidicola auf Erdnusspflanzen*

a) *Residual-protektive Wirkung*

10-15 cm hohe Erdnusspflanzen wurden mit einer aus Spritzpulver der Wirksubstanz hergestellten Spritzbrühe (0,002% Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

b) *Systemische Wirkung*

Zu 10-15 cm hohen Erdnusspflanzen wurde eine aus Spritzpulver des Wirkstoffes hergestellte Spritzbrühe gegossen (0,06% Aktivsubstanz bezogen auf das Erdvolumen). Nach 48 Stunden wurden die behandelten Pflanzen mit einer Konidiensuspension des Pilzes infiziert und 72 Stunden bei ca. 21°C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100%), zeigten Erdnusspflanzen, die mit Wirkstoffen aus den Tabellen behandelt wurden, einen stark reduzierten Cercospora-Befall. So verhinderten die Verbindungen in obigen Versuchen das Auftreten von Flecken fast vollständig (0-10%).

*Beispiel 3.3:*

*Wirkung gegen Erysiphae graminis auf Gerste*

a) *Residual-protektive Wirkung*

Ca. 8 cm hohe Gerstenpflanzen wurden mit einer aus Spritzpulver des Wirkstoffes hergestellten Spritzbrühe (0,006% Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) *Systemische Wirkung*

Zu ca. 8 cm hohen Gerstenpflanzen wurde eine aus Spritzpulver des Wirkstoffes hergestellte Spritzbrühe gegossen (0,002% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100%. Unter anderen Verbindungen aus den Tabellen hemmten die Verbindungen Nr. 1.4, 1.7 und 1.18 den Pilzbefall bei Gerste auf 0 bis 5%.

*Beispiel 3.4:*

*Residual-protektive Wirkung gegen Venturia inaequalis auf Apfeltrieben.*

Apfelstecklinge mit 10-20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen aus den Tabellen 1 bis 8 bewirkten eine deutliche Hemmung des Krankheitsbefalls. Unbehandelte aber infizierte Triebe zeigten einen 100%igen Venturia-Befall.

*Beispiel 3.5:*

*Wirkung gegen Botrytis cinerea auf Bohnen*
*Residual protektive Wirkung*

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100% relativer Luftfeuchtigkeit und 21°C erfolgte die Beurteilung des Pilzbefalls. Die Verbindungen aus den Tabellen 1 bis 8 hemmten in vielen Fällen die Pilzinfektion sehr stark. Bei einer Konzentration von 0,02% erwiesen sich z.B. die Verbindungen aus den Tabellen als sehr wirksam. Der Botrytis-Befall unbehandelter aber infizierter Bohnenpflanzen betrug 100%.

*Beispiel 3.6:*

*Wirkung gegen Piricularia oryzae auf Reispflanzen*
*Residual-protektive Wirkung*

Reispflanzen wurden nach zweiwöchiger Anzucht mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95-100% relativer Luftfeuchtigkeit und 24°C wurde der Pilzbefall beurteilt.

Reispflanzen, die mit einer Spritzbrühe behandelt wurden, die als Aktivsubstanz die Verbindung 1.4 enthielt, zeigten im Vergleich zu unbehandelten Kontrollpflanzen (100% Befall) weniger als 10% Pilzbefall.

**Patentansprüche für die Vertragsstaaten:**
DE, GB, FR, CH, LI, IT, NL, BE, SE, LU

1. Verbindungen der Formel I

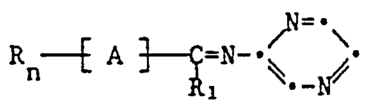
(I)

worin

A für Phenyl, Naphthyl, Biphenyl, Phenoxyphenyl oder Phenylthiophenyl steht;

R Halogen, Cyano, Nitro, $C_1$-$C_4$-Alkyl, $C_1$-$C_4$-Alkoxy, $C_1$-$C_4$-Haloalkoxy, $C_1$-$C_4$-Haloalkyl oder Di($C_1$-$C_4$-alkyl)amino bedeutet;

n für 0, 1, 2, 3, 4 oder 5 steht;

$R_1$ eine der Gruppen $OR_2$, $SR_2$ oder $N(R_3)(R_4)$ repräsentiert, wobei $R_2$ für $C_1$-$C_8$-Alkyl, $C_1$-$C_4$-Haloalkyl, $C_3$-$C_8$-Alkenyl, $C_3$-$C_8$-Alkinyl oder für einen unsubstituierten oder ein- bis dreifach durch R substituierten Rest aus der Reihe Phenyl, Benzyl, $C_3$-$C_7$-Cycloalkyl oder Furfuryl steht; und

$R_3$ und $R_4$ unabhängig voneinander für Wasserstoff oder $C_1$-$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder als ein weiteres Heteroatom N, O oder S enthält.

2. Verbindungen der Formel I nach Anspruch 1, dadurch gekennzeichnet, dass

A für Phenyl, Naphthyl, Biphenyl, Phenoxyphenyl oder Phenylthiophenyl steht;

R Halogen, Cyano, Nitro, $C_1$-$C_4$-Alkyl, $C_1$-$C_4$-Alkoxy, $C_1$-$C_4$-Haloalkoxy, $C_1$-$C_4$-Haloalkyl oder Di($C_1$-$C_4$-alkyl)amino bedeutet;

n für 0, 1, 2, 3, 4 oder 5 steht;

$R_1$ eine der Gruppen $OR_2$, $SR_2$ oder $N(R_3)(R_4)$ repräsentiert, wobei $R_2$ für $C_1$-$C_8$-Alkyl, $C_3$-$C_8$-Alkenyl, $C_3$-$C_8$-Alkinyl oder für einen $C_1$-$C_4$-Haloalkyl, unsubstituierten oder ein- bis dreifach durch R substituierten Rest aus der Reihe Phenyl, Benzyl, $C_3$-$C_7$-Cycloalkyl oder Furfuryl steht; und

$R_3$ und $R_4$ unabhängig voneinander für Wasserstoff oder $C_1$-$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder als ein weiteres Heteroatom N, O oder S enthält.

3. Verbindungen der Formel I nach Anspruch 1, dadurch gekennzeichnet, dass A für Phenyl steht; R Halogen, Cyano, Methyl, Ethyl, Methoxy, Ethoxy, $CF_3$, $C_1$-$C_2$-Haloalkoxy, $N(CH_3)_2$ oder $N(C_2H_5)_2$ bedeutet; n für 1, 2 oder 3 steht; $R_1$ eine der Gruppen $OR_2$ oder $SR_2$ repräsentiert; wobei $R_2$ für $C_1$-$C_8$-Alkyl, $C_3$-$C_8$-Alkenyl, $C_3$-$C_8$-Alkinyl oder für einen unsubstituierten oder ein- bis dreifach durch Halogen, Cyano, Methyl, Ethyl, Methoxy, Ethoxy, $CF_3$, $C_1$-$C_2$-Haloalkoxy oder $N(C_1$-$C_2$-$Alkyl)_2$ substituierten Rest aus der Reihe Phenyl, Benzyl, $C_5$-$C_6$-Cycloalkyl und Furfuryl steht.

4. Verbindungen der Formel I nach Anspruch 3, dadurch gekennzeichnet, dass A für Phenyl steht; R Fluor, Chlor, Brom, Methyl, Methoxy oder $CF_3$ bedeutet; n für 1 oder 2 steht; $R_1$ für $OR_2$ oder $SR_2$ steht; wobei $R_2$ für $C_1$-$C_5$-Alkyl, $C_3$-$C_4$-Alkenyl, $C_3$-$C_4$-Alkinyl, Cyclopentyl, Cyclohexyl, Furfuryl oder einen unsubstituierten oder ein- bis dreifach durch Fluor, Chlor, Brom, Methyl, Methoxy oder $CF_3$ substituierten Rest aus der Reihe Phenyl und Benzyl bedeutet.

5. Verbindungen der Formel I nach Anspruch 4, dadurch gekennzeichnet, dass R für Fluor oder Chlor steht; n die 2- und 4-Position des Phenylrings repräsentiert; $R_1$ für $OR_2$ oder SR steht; wobei $R_2$ für $C_1$-$C_5$-Alkyl, Allyl, Propargyl, Cyclohexyl, Benzyl oder unsubstituiertes oder ein- bis dreifach durch Fluor, Chlor, Brom, Methyl, Methoxy oder $CF_3$ substituiertes Phenyl bedeutet.

6. Verbindungen der Formel I nach Anspruch 1, dadurch gekennzeichnet, dass A für Phenoxyphenyl steht; R Halogen, Cyano, Methyl, Ethyl, Methoxy, Ethoxy, $CF_3$, $C_1$-$C_2$-Haloalkoxy, $N(CH_3)_2$ oder $N(C_2H_5)_2$ bedeutet, n für 0, 1, 2, 3, 4 oder 5 steht; $R_1$ eine der Gruppen $OR_2$ oder $SR_2$ repräsentiert; wobei $R_2$ für $C_1$-$C_8$-Alkyl, $C_3$-$C_8$-Alkenyl, $C_3$-$C_8$-Alkinyl oder für einen unsubstituierten oder ein- bis dreifach durch Halogen, Cyano, Methyl, Ethyl, Methoxy, Ethoxy, $CF_3$, $C_1$-$C_2$-Haloalkoxy oder $N(C_1$-$C_2$-$Alkyl)_2$ substituierten Rest aus der Reihe Phenyl, Benzyl, $C_5$-$C_6$-Cycloalkyl oder Furfuryl steht.

7. Verbindungen der Formel I nach Anspruch 6, dadurch gekennzeichnet, dass A für (p-Phenoxy)-phenyl steht.

8. Verbindungen der Formel I nach Anspruch 7, dadurch gekennzeichnet, dass A für (p-Phenoxy)-phenyl steht; R Fluor, Chlor, Brom, Methyl, Methoxy oder $CF_3$ bedeutet; n für 0, 1, 2, 3 oder 4 steht; $R_1$ für $OR_2$ oder $SR_2$ steht; wobei $R_2$ für $C_1$-$C_5$-Alkyl, $C_3$-$C_4$-Alkenyl, $C_3$-$C_4$-Alkinyl, Cyclopentyl, Cyclohexyl, Furfuryl oder einen unsubstituierten oder ein- bis dreifach durch Fluor, Chlor, Brom, Methyl, Methoxy oder $CF_3$ substituierten Rest aus der Reihe Phenyl und Benzyl bedeutet.

9. Verbindungen der Formel I nach Anspruch 8, dadurch gekennzeichnet, dass A für (p-Phenoxy)phenyl steht; R Fluor, Chlor, Methyl, Methoxy oder $CF_3$ bedeutet; n für 0, 1, 2 oder 3 steht; $R_1$ für $OR_2$ oder $SR_2$ steht; wobei $R_2$ für $C_1$-$C_5$-Alkyl, Allyl, Propargyl, Cyclohexyl, Benzyl oder unsubstituiertes oder ein- bis dreifach durch Fluor, Chlor, Brom, Methyl, Methoxy oder $CF_3$ substituiertes Phenyl steht.

10. Eine Verbindung der Formel I nach Anspruch 1, dadurch gekennzeichnet, dass sie ausgewählt ist aus der Reihe:

[2,4-Dichlorbenzyl-C-(tert.butylthio)imino]pyrazin (Nr. 1.7);

[2,4-Dichlorbenzyl-C-(isopropylthio)imino]pyrazin (Nr. 1.4).

11. Verfahren zur Herstellung von Verbindungen der Formel I, dadurch gekennzeichnet, dass man eine Verbindung der Formel II

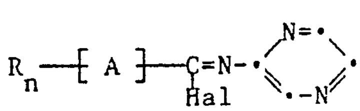
(II)

in Gegenwart einer Base mit einer Verbindung der Formel III

$R_1$-H (III)

umsetzt; dabei haben $R_n$, A und $R_1$ in den Formeln II und III die unter Formel I angegebenen Bedeutungen, Hal steht für Halogen, vorzugsweise für Chlor oder Brom.

12. Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I gemäss Anspruch 1 enthält.

13. Mittel gemäss Anspruch 12, dadurch gekennzeichnet, dass es als mindestens eine aktive Komponente eine Verbindung der Formel I gemäss Anspruch 3 enthält.

14. Mittel zur Bekämpfung von Mikroorganismen gemäss Anspruch 12, dadurch gekennzeichnet, dass es als mindestens eine aktive Komponente eine Verbindung der Formel I gemäss Anspruch 4 enthält.

15. Mittel zur Bekämpfung von Mikroorganismen gemäss Anspruch 12, dadurch gekennzeichnet, dass es als mindestens eine aktive Komponente eine Verbindung der Formel I gemäss einem der Ansprüche 5 bis 10 enthält.

16. Mittel nach einem der Ansprüche 12 bis 15, dadurch gekennzeichnet, dass es 0,1 bis 99% eines Wirkstoffs der Formel I, 99,9 bis 1% eines festen oder flüssigen Zusatzstoffes und 0 bis 25% eines Tensides enthält.

17. Mittel nach Anspruch 16, dadurch gekennzeichnet, dass es 0,1 bis 95% eines Wirkstoffes der Formel I, 99,8 bis 5% eines festen oder flüssigen Zusatzstoffes und 0,1 bis 25% eines Tensides enthält.

18. Verfahren zur Herstellung eines agrochemischen Mittels, dadurch gekennzeichnet, dass man mindestens eine Verbindung der Formel I gemäss einem der Ansprüche 12 bis 17 mit geeigneten festen oder flüssigen Zusatzstoffen und Tensiden innig vermischt.

19. Verfahren zur Bekämpfung oder Verhütung eines Befalls von Kulturpflanzen durch phytopathogene Mikroorganismen, dadurch gekennzeichnet, dass man eine Verbindung der Formel I auf die Pflanze oder deren Standort appliziert.

20. Verwendung von Verbindungen der Formel I zur Bekämpfung von Pflanzenkrankheiten, die auf Mikroorganismen zurückzuführen sind.

21. Verwendung gemäss Anspruch 20 von Verbindungen der Formel I gemäss einem der Ansprüche 3 bis 10.

22. Verwendung gemäss Anspruch 20, dadurch gekennzeichnet, dass es sich bei den Mikroorganismen um phytopathogene Pilze handelt.

23. Verwendung gemäss Anspruch 22 gegen Pilze aus den Klassen Ascomycetes, Basidiomycetes oder Fungi imperfecti.

24. Verbindungen der Formel II

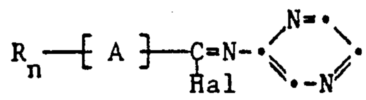 (II),

worin $R_n$ und A die unter Formel I angegebenen Bedeutungen haben und Hal für Halogen, vorzugsweise Chlor oder Brom steht.

25. Verbindungen der Formel VI

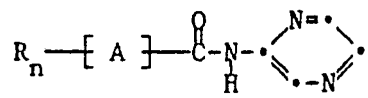 (VI),

worin $R_n$ und A die unter Formel I angegebenen Bedeutungen haben.

**Patentansprüche für den Vertragsstaat:** AT

1. Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I enthält,

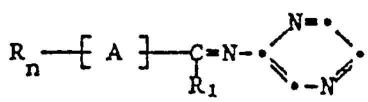 (I)

worin

A für Phenyl, Naphthyl, Biphenyl, Phenoxyphenyl oder Phenylthiophenyl steht;
R Halogen, Cyano, Nitro, $C_1$-$C_4$-Alkyl, $C_1$-$C_4$-Alkoxy, $C_1$-$C_4$-Haloalkoxy, $C_1$-$C_4$-Haloalkyl oder Di($C_1$-$C_4$-alkyl)amino bedeutet;
n für 0, 1, 2, 3, 4 oder 5 steht;
$R_1$ eine der Gruppen $OR_2$, $SR_2$ oder $N(R_3)(R_4)$ repräsentiert, wobei $R_2$ für $C_1$-$C_8$-Alkyl, $C_1$-$C_4$-Haloalkyl, $C_3$-$C_8$-Alkenyl, $C_3$-$C_8$-Alkinyl oder für einen unsubstituierten oder ein- bis dreifach durch R substituierten Rest aus der Reihe Phenyl, Benzyl, $C_3$-$C_7$-Cycloalkyl oder Furfuryl steht; und
$R_3$ und $R_4$ unabhängig voneinander für Wasserstoff oder $C_1$-$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder als ein weiteres Heteroatom N, O oder S enthält.

2. Schädlingsbekämpfungsmittel gemäss Anspruch 1, dadurch gekennzeichnet, dass

A für Phenyl, Naphthyl, Biphenyl, Phenoxyphenyl oder Phenylthiophenyl steht;
R Halogen, Cyano, Nitro, $C_1$-$C_4$-Alkyl, $C_1$-$C_4$-Alkoxy, $C_1$-$C_4$-Haloalkoxy, $C_1$-$C_4$-Haloalkyl oder Di($C_1$-$C_4$-alkyl)amino bedeutet;
n für 0, 1, 2, 3, 4 oder 5 steht;
$R_1$ eine der Gruppen $OR_2$, $SR_2$ oder $N(R_3)(R_4)$ repräsentiert, wobei $R_2$ für $C_1$-$C_8$-Alkyl, $C_3$-$C_8$-Alke-

nyl, $C_3$-$C_8$-Alkinyl oder für einen $C_1$-$C_4$-Haloalkyl, unsubstituierten oder ein- bis dreifach durch R substituierten Rest aus der Reihe Phenyl, Benzyl, $C_3$-$C_7$-Cycloalkyl oder Furfuryl steht; und

$R_3$ und $R_4$ unabhängig voneinander für Wasserstoff oder $C_1$-$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder als ein weiteres Heteroatom N, O oder S enthält.

3. Schädlingsbekämpfungsmittel gemäss Anspruch 1, dadurch gekennzeichnet, dass A für Phenyl steht; R Halohen, Cyano, Methyl, Ethyl, Methoxy, Ethoxy, $CF_3$, $C_1$-$C_2$-Haloalkoxy, $N(CH_3)_2$ oder $N(C_2H_5)_2$ bedeutet; n für 1, 2 oder 3 steht; $R_1$ eine der Gruppen $OR_2$ oder $SR_2$ repräsentiert; wobei $R_2$ für $C_1$-$C_8$-Alkyl, $C_3$-$C_8$-Alkenyl, $C_3$-$C_8$-Alkinyl oder für einen unsubstituierten oder ein- bis dreifach durch Halogen, Cyano, Methyl, Ethyl, Methoxy, Ethoxy, $CF_3$, $C_1$-$C_2$-Haloalkoxy oder $N(C_1$-$C_2$-$Alkyl)_2$ substituierten Rest aus der Reihe Phenyl, Benzyl, $C_5$-$C_6$-Cycloalkyl und Furfuryl steht.

4. Schädlingsbekämpfungsmittel gemäss Anspruch 3, dadurch gekennzeichnet, dass A für Phenyl steht; R Fluor, Chlor, Brom, Methyl, Methoxy oder $CF_3$ bedeutet; n für 1 oder 2 steht; $R_1$ für $OR_2$ oder $SR_2$ steht; wobei $R_2$ für $C_1$-$C_5$-Alkyl, $C_3$-$C_4$-Alkenyl, $C_3$-$C_4$-Alkinyl, Cyclopentyl, Cyclohexyl, Furfuryl oder einen unsubstituierten oder ein- bis dreifach durch Fluor, Chlor, Brom, Methyl, Methoxy oder $CF_3$ substituierten Rest aus der Reihe Phenyl und Benzyl bedeutet.

5. Schädlingsbekämpfungsmittel gemäss Anspruch 4, dadurch gekennzeichnet, dass R für Fluor oder Chlor steht; n die 2- und 4-Position des Phenylrings repräsentiert; $R_1$ für $OR_2$ oder $SR_2$ steht; wobei $R_2$ für $C_1$-$C_5$-Alkyl, Allyl, Propargyl, Cyclohexyl, Benzyl oder unsubstituiertes oder ein-bis dreifach durch Fluor, Chlor, Brom, Methyl, Methoxy oder $CF_3$ substituiertes Phenyl bedeutet.

6. Schädlingsbekämpfungsmittel gemäss Anspruch 1, dadurch gekennzeichnet, dass A für Phenoxyphenyl steht; R Halogen, Cyano, Methyl, Ethyl, Methoxy, Ethoxy, $CF_3$, $C_1$-$C_2$-Haloalkoxy, $N(CH_3)_2$ oder $N(C_2H_5)_2$ bedeutet, n für 0, 1, 2, 3, 4 oder 5 steht; $R_1$ eine der Gruppen $OR_2$ oder $SR_2$ repräsentiert; wobei $R_2$ für $C_1$-$C_8$-Alkyl, $C_3$-$C_8$-Alkenyl, $C_3$-$C_8$-Alkinyl oder für einen unsubstituierten oder ein- bis dreifach durch Halogen, Cyano, Methyl, Ethyl, Methoxy, Ethoxy, $CF_3$, $C_1$-$C_2$-Haloalkoxy oder $N(C_1$-$C_2$-$Alkyl)_2$ substituierten Rest aus der Reihe Phenyl, Benzyl, $C_5$-$C_6$-Cycloalkyl oder Furfuryl steht.

7. Schädlingsbekämpfungsmittel gemäss Anspruch 6, dadurch gekennzeichnet, dass A für (p-Phenoxy)-phenyl steht.

8. Schädlingsbekämpfungsmittel gemäss Anspruch 7, dadurch gekennzeichnet, dass A für (p-Phenoxy)phenyl steht; R Fluor, Chlor, Brom, Methyl, Methoxy oder $CF_3$ bedeutet; n für 0, 1, 2, 3 oder 4 steht; $R_1$ für $OR_2$ oder $SR_2$ steht; wobei $R_2$ für $C_1$-$C_5$-Alkyl, $C_3$-$C_4$-Alkenyl, $C_3$-$C_4$-Alkinyl, Cyclopentyl, Cyclohexyl, Furfuryl oder einen unsubstituierten oder ein- bis dreifach durch Fluor, Chlor, Brom, Methyl, Methoxy oder $CF_3$ substituierten Rest aus der Reihe Phenyl und Benzyl bedeutet.

9. Schädlingsbekämpfungsmittel gemäss Anspruch 8, dadurch gekennzeichnet, dass A für (p-Phenoxy)phenyl steht; R Fluor, Chlor, Methyl, Methoxy oder $CF_3$ bedeutet; n für 0, 1, 2 oder 3 steht; $R_1$ für $OR_2$ oder $SR_2$ steht; wobei $R_2$ für $C_1$-$C_5$-Alkyl, Allyl, Propargyl, Cyclohexyl, Benzyl oder unsubstituiertes oder ein- bis dreifach durch Fluor, Chlor, Brom, Methyl, Methoxy oder $CF_3$ substituiertes Phenyl steht.

10. Schädlingsbekämpfungsmittel gemäss Anspruch 1, dadurch gekennzeichnet, dass sie ausgewählt ist aus der Reihe:
[2,4-Dichlorbenzyl-C-(tert.butylthio)imino]pyrazin (Nr. 1.7);
[2,4-Dichlorbenzyl-C-(isopropylthio)imino]pyrazin (Nr. 1.4).

11. Schädlingsbekämpfungsmittel gemäss einem der Ansprüche 1 bis 10, dadurch gekennzeichnet, dass es 0,1 bis 99% eines Wirkstoffs der Formel I, 99,9 bis 1% eines festen oder flüssigen Zusatzstoffes und 0 bis 25% eines Tensides enthält.

12. Mittel nach Anspruch 11, dadurch gekennzeichnet, dass es 0,1 bis 95% eines Wirkstoffes der Formel I, 99,8 bis 5% eines festen oder flüssigen Zusatzstoffes und 0,1 bis 25% eines Tensides enthält.

13. Verfahren zur Herstellung von Verbindungen der Formel I, dadurch gekennzeichnet, dass man eine Verbindung der Formel II

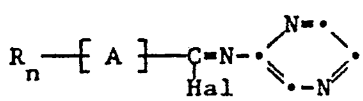 (II)

in Gegenwart einer Base mit einer Verbindung der Formel III

$$R_1\text{-}H \quad \text{(III)}$$

umsetzt; dabei haben $R_n$, A und $R_1$ in den Formeln II und III die unter Formel I angegebenen Bedeutungen. Hal steht für Halogen, vorzugsweise für Chlor oder Brom.

14. Verfahren zur Herstellung eines agrochemischen Mittels, dadurch gekennzeichnet, dass man mindestens eine Verbindung der Formel I gemäss einem der Ansprüche 1 bis 12 mit geeigneten festen oder flüssigen Zusatzstoffen und Tensiden innig vermischt.

15. Verfahren zur Bekämpfung oder Verhütung eines Befalls von Kulturpflanzen durch phytopathogene Mikroorganismen, dadurch gekennzeichnet, dass man eine Verbindung der Formel I auf die Pflanze oder deren Standort appliziert.

16. Verwendung von Verbindungen der Formel I zur Bekämpfung von Pflanzenkrankheiten, die auf Mikroorganismen zurückzuführen sind.

17. Verwendung gemäss Anspruch 16 von Verbindungen der Formel I gemäss einem der Ansprüche 3 bis 10.

18. Verwendung gemäss Anspruch 16, dadurch gekennzeichnet, dass es sich bei den Mikroorganismen um phytopathogene Pilze handelt.

19. Verwendung gemäss Anspruch 18 gegen Pilze aus den Klassen Ascomycetes, Basidiomycetes oder Fungi imperfecti.

**Claims for the Contracting States:**
DE, GB, FR, CH, LI, IT, NL, BE, SE, LU

1. Compounds of formula I

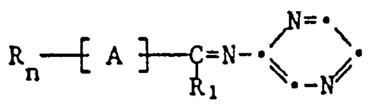
(I)

wherein

A is phenyl, naphthyl, biphenyl, phenoxyphenyl or phenylthiophenyl;

R is halogen, cyano, nitro, $C_1$-$C_4$alkyl, $C_1$-$C_4$-alkoxy, $C_1$-$C_4$haloalkoxy, $C_1$-$C_4$haloalkyl or di($C_1$-$C_4$alkyl)amino;

n is 0, 1, 2, 3, 4 or 5;

$R_1$ is one of the groups $OR_2$, $SR_2$ and $N(R_3)(R_4)$, wherein $R_2$ is $C_1$-$C_8$alkyl, $C_1$-$C_4$haloalkyl, $C_3$-$C_8$-alkenyl or $C_3$-$C_8$alkynyl, or is a radical from the series phenyl, benzyl, $C_3$-$C_7$cycloalkyl and furfuryl which is unsubstituted or mono- to tri-substituted by R; and

$R_3$ and $R_4$ independently of each other are hydrogen or $C_1$-$C_4$alklyl or, together with the amine nitrogen atom, form a saturated five- or six-membered heterocycle which contains as hetero atom either only the amine nitrogen atom or, as a further hetero atom, N, O or S.

2. Compounds of formula I according to claim 1, characterised in that

A is phenyl, naphthyl, biphenyl, phenoxyphenyl or phenylthiophenyl;

R is halogen, cyano, nitro, $C_1$-$C_4$alkoxy, $C_1$-$C_4$haloalkoxy, $C_1$-$C_4$haloalkyl or di($C_1$-$C_4$alkyl)-amino;

n is 0, 1, 2, 3, 4 or 5;

$R_1$ is one of the groups $OR_2$, $SR_2$ and $N(R_3)(R_4)$, wherein $R_2$ is $C_1$-$C_8$alkyl, $C_3$-$C_8$alkenyl, $C_3$-$C_8$alkynyl or $C_1$-$C_4$haloalkyl, or is a radical from the series phenyl, benzyl, $C_3$-$C_7$cycloalkyl and furfuryl which is unsubstituted or mono- to tri-substituted by R; and

$R_3$ and $R_4$ independently of each other are hydrogen or $C_1$-$C_4$alkyl or, together with the amine nitrogen atom, form a saturated five- or six-membered heterocycle which contains as hetero atom either only the amine nitrogen atom or, as a further hetero atom, N, O or S.

3. Compounds of formula I according to claim 1, characterised in that A is phenyl; R is halogen, cyano, methyl, ethyl, methoxy, ethoxy, $CF_3$, $C_1$-$C_2$-haloalkoxy, $N(CH_3)_2$ or $N(C_2H_5)_2$; n is 1, 2 or 3; $R_1$ is one of the groups $OR_2$ and $SR_2$; wherein $R_2$ is $C_1$-$C_8$alkyl, $C_3$-$C_8$alkenyl or $C_3$-$C_8$alkynyl, or is a radical from the series phenyl, benzyl, $C_5$-$C_6$cycloalkyl and furfuryl which is unsubstituted or mono- to trisubstituted by halogen, cyano, methyl ethyl, methoxy, ethoxy, $CF_3$, $C_1$-$C_2$haloalkoxy or $N(C_1$-$C_2alkyl)_2$.

4. Compounds of formula I according to claim 3, characterised in that A is phenyl; R is fluorine, chlorine, bromine, methyl, methoxy or $CF_3$; n is 1 or 2; $R_1$ is $OR_2$ or $SR_2$; wherein $R_2$ is $C_1$-$C_5$alkyl, $C_3$-$C_4$alkenyl, $C_3$-$C_4$alkynyl, cyclopentyl, cyclohexyl or furfuryl, or is a radical from the series phenyl and benzyl which is unsubstituted or mono- to tri-substituted by fluorine, chlorine, bromine, methyl, methoxy or $CF_3$.

5. Compounds of formula I according to claim 4, characterised in that R is fluorine or chlorine; n represents the 2- and 4-positions of the phenyl ring; $R_1$ is $OR_2$ or $SR_2$; wherein $R_2$ is $C_1$-$C_5$alkyl, allyl, propargyl, cyclohexyl or benzyl, or is phenyl which is unsubstituted or mono- to trisubstituted by fluorine, chlorine, bromine, methyl, methoxy or $CF_3$.

6. Compounds of formula I according to claim 1, characterised in that A is phenoxyphenyl; R is halogen, cyano, methyl, ethyl, methoxy, ethoxy, $CF_3$, $C_1$-$C_2$haloalkoxy, $N(CH_3)_2$ or $N(C_2H_5)_2$; n is 0, 1, 2, 3, 4 or 5; $R_1$ is one of the groups $OR_2$ and $SR_2$; wherein $R_2$ is $C_1$-$C_8$alkyl, $C_3$-$C_8$alkenyl or $C_3$-$C_8$alkynyl, or is a radical from the series phenyl, benzyl, $C_5$-$C_6$cycloalkyl and furfuryl which is unsubstituted or mono- to tri-substituted by halogen, cyano, methyl, ethyl, methoxy, ethoxy, $CF_3$, $C_1$-$C_2$haloalkoxy or $N(C_1$-$C_2alkyl)_2$.

7. Compounds of formula I according to claim 6, characterised in that A is (p-phenoxy)phenyl.

8. Compounds of formula I according to claim 7, characterised in that A is (p-phenoxy)phenyl; R is fluorine, chlorine, bromine, methyl, methoxy or $CF_3$; n is 0, 1, 2, 3 or 4; $R_1$ is $OR_2$ or $SR_2$; wherein $R_2$ is $C_1$-$C_5$alkyl, $C_3$-$C_4$alkenyl, $C_3$-$C_4$alkynyl, cyclopentyl, cyclohexyl or furfuryl, or is a radical from the series phenyl and benzyl which is unsubstituted or is mono- to tri-substituted by fluorine, chlorine, bromine, methyl, methoxy or $CF_3$.

9. Compounds of formula I according to claim 8, characterised in that A is (p-phenoxy)phenyl; R is fluorine, chlorine, methyl, methoxy or $CF_3$; n is 0, 1, 2 or 3; $R_1$ is $OR_2$ or $SR_2$; wherein $R_2$ is $C_1$-$C_5$alkyl, allyl, propargyl, cyclohexyl or benzyl, or is phenyl which is unsubstituted or mono- to tri-substituted by fluorine, chlorine, bromine, methyl, methoxy or $CF_3$.

10. A compound of formula I according to claim 1, characterised in that it is selected from the series:

[2,4-dichlorobenzyl-C-(tert.-butylthio)imino]pyrazine (no. 1.7);

[2,4-dichlorobenzyl-C-(isopropylthio)imino]pyrazine (no. 1.4).

11. A process for the preparation of compounds of formula I, characterised in that a compound of formula II

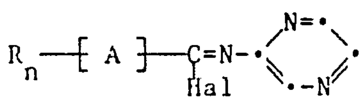 (II)

is reacted in the presence of a base with a compound of formula III

$R_1H$ (III),

$R_n$, A and $R_1$ in formulae II and III being as defined for formula I, and Hal being halogen, preferably chlorine or bromine.

12. A pesticidal composition for controlling or preventing an attack by microorganisms, characterised in that it contains, as at least one active component, a compound of formula I according to claim 1.

13. A composition according to claim 12, characterised in that it contains, as at least one active component, a compound of formula I according to claim 3.

14. A composition for controlling microorganisms according to claim 12, characterised in that it contains, as at least one active component, a compound of formula I according to claim 4.

15. A composition for controlling microorganisms according to claim 12, characterised in that it contains, as at least one active component, a compound of formula I according to any one of claims 5 to 10.

16. A composition according to any one of claims 12 to 15, characterised in that it contains from 0.1 to 99% of an active ingredient of formula I, from 99.9 to 1% of a solid or liquid adjuvant and from 0 to 25% of a surfactant.

17. A composition according to claim 16, characterised in that it contains from 0.1 to 95% of an active ingredient of formula I, from 99.8 to 5% of a solid or liquid adjuvant and from 0.1 to 25% of a surfactant.

18. A process for the preparation of an agrochemical composition, characterised in that at least one compound of formula I according to any one of claims 12 to 17 is homogeneously mixed with suitable solid or liquid adjuvants and surfactants.

19. A method of controlling or preventing an attack on cultivated plants by phytopathogenic microorganisms, characterised in that a compound of formula I is applied to the plant or to the locus thereof.

20. The use of compounds of formula I for controlling plant diseases that are attributable to microorganisms.

21. The use according to claim 20 of compounds of formula I according to any one of claims 3 to 10.

22. The use according to claim 20, characterised in that the microorganisms are phytopathogenic fungi.

23. The use according to claim 22 against fungi from the classes Ascomycetes, Basidiomycetes or Fungi imperfecti.

24. Compounds of formula II

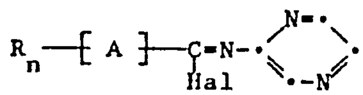 (II)

wherein $R_n$ and A are as defined for formula I and Hal is halogen, preferably chlorine or bromine.

25. Compounds of formula VI

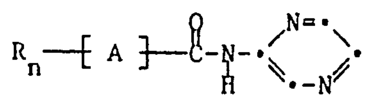 (VI)

wherein $R_n$ and A are as defined for formula I.

**Claims for the Contracting State:** AT

1. A pesticidal composition for controlling or preventing an attack by microorganisms, characterised in that it contains, as at least one active component, a compound of formula I

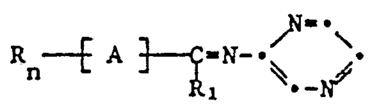 (I)

wherein

A is phenyl, naphthyl, biphenyl, phenoxyphenyl or phenylthiophenyl;
R is halogen, cyano, nitro, $C_1$-$C_4$alkyl, $C_1$-$C_4$-alkoxy, $C_1$-$C_4$haloalkoxy, $C_1$-$C_4$haloalkyl or di($C_1$-$C_4$alkyl)amino;
n is 0, 1, 2, 3, 4 or 5;
$R_1$ is one of the groups $OR_2$, $SR_2$ and $N(R_3)(R_4)$, wherein $R_2$ is $C_1$-$C_8$alkyl, $C_1$-$C_4$haloalkyl, $C_3$-$C_8$alkenyl or $C_3$-$C_8$alkynyl, or is a radical from the series phenyl, benzyl, $C_3$-$C_7$cycloalkyl and furfuryl which is unsubstituted or mono- to tri-substituted by R; and
$R_3$ and $R_4$ independently of each other are hydrogen or $C_1$-$C_4$alkyl or, together with the amine nitrogen atom, form a saturated five- or six-membered heterocycle which contains as hetero atom either only the amine nitrogen atom or, as a further hetero atom, N, O or S.

2. A pesticidal composition according to claim 1, characterised in that
A is phenyl, naphthyl, biphenyl, phenoxyphenyl or phenylthiophenyl;
R is halogen, cyano, nitro, $C_1$-$C_4$alkyl, $C_1$-$C_4$alkoxy, $C_1$-$C_4$haloalkoxy $C_1$-$C_4$haloalkyl or di($C_1$-$C_4$alkyl)amino;
n is 0, 1, 2, 3, 4 or 5;
$R_1$ is one of the groups $OR_2$, $SR_2$ and $N(R_3)(R_4)$, wherein $R_2$ is $C_1$-$C_8$alkyl, $C_3$-$C_8$alkenyl, $C_3$-$C_8$alkynyl

or $C_1$-$C_4$haloalkyl, or is a radical from the series phenyl, benzyl, $C_3$-$C_7$cycloalkyl and furfuryl which is unsubstituted or mono- to tri-substituted by R; and

$R_3$ and $R_4$ independently of each other are hydrogen or $C_1$-$C_4$alkyl or, together with the amine nitrogen atom, form a saturated five- or six-membered heterocycle which contains as hetero atom either only the amine nitrogen atom or, as a further hetero atom, N, O or S.

3. A pesticidal composition according to claim 1, characterised in that A is phenyl; R is halogen, cyano, methyl, ethyl, methoxy, ethoxy, $CF_3$, $C_1$-$C_2$haloalkoxy, $N(CH_3)_2$ or $N(C_2H_5)_2$; n is 1, 2 or 3; $R_1$ is one of the groups $OR_2$ and $SR_2$; wherein $R_2$ is $C_1$-$C_8$alkyl, $C_3$-$C_8$alkenyl or $C_3$-$C_8$alkynyl, or is a radical from the series phenyl, benzyl, $C_5$-$C_6$cycloalkyl and furfuryl which is unsubstituted or mono- to trisubstituted by halogen, cyano, methyl ethyl, methoxy, ethoxy, $CF_3$, $C_1$-$C_2$haloalkoxy or $N(C_1$-$C_2alkyl)_2$.

4. A pesticidal composition according to claim 3, characterised in that A is phenyl; R is fluorine, chlorine, bromine, methyl, methoxy or $CF_3$; n is 1 or 2; $R_1$ is $OR_2$ or $SR_2$; wherein $R_2$ is $C_1$-$C_5$alkyl, $C_3$-$C_4$alkenyl, $C_3$-$C_4$alkynyl, cyclopentyl, cyclohexyl or furfuryl, or is a radical from the series phenyl and benzyl which is unsubstituted or mono- to tri-substituted by fluorine, chlorine, bromine, methyl, methoxy or $CF_3$.

5. A pesticidal composition according to claim 4, characterised in that R is fluorine or chlorine; n represents the 2- and 4-positions of the phenyl ring; $R_1$ is $OR_2$ or $SR_2$; wherein $R_2$ is $C_1$-$C_5$alkyl, allyl, propargyl, cyclohexyl or benzyl, or is phenyl which is unsubstituted or mono- to trisubstituted by fluorine, chlorine, bromine, methyl, methoxy or $CF_3$.

6. A pesticidal composition according to claim 1, characterised in that A is phenoxyphenyl; R is halogen, cyano, methyl, ethyl, methoxy, ethoxy, $CF_3$, $C_1$-$C_2$haloalkoxy, $N(CH_3)_2$ or $N(C_2H_5)_2$; n is 0, 1, 2, 3, 4 or 5; $R_1$ is one of the groups $OR_2$ and $SR_2$; wherein $R_2$ is $C_1$-$C_8$alkyl, $C_3$-$C_8$alkenyl or $C_3$-$C_8$alkynyl, or is a radical from the series phenyl, benzyl, $C_5$-$C_6$cycloalkyl and furfuryl which is unsubstituted or mono- to tri-substituted by halogen, cyano, methyl, ethyl, methoxy, ethoxy, $CF_3$, $C_1$-$C_2$haloalkoxy or $N(C_1$-$C_2alkyl)_2$.

7. A pesticidal composition according to claim 6, characterised in that A is (p-phenoxy)phenyl.

8. A pesticidal composition according to claim 7, characterised in that A is (p-phenoxy)phenyl; R is fluorine, chlorine, bromine, methyl, methoxy or $CF_3$; n is 0, 1, 2, 3 or 4; $R_1$ is $OR_2$ or $SR_2$; wherein $R_2$ is $C_1$-$C_5$alkyl, $C_3$-$C_4$alkenyl, $C_3$-$C_4$alkynyl, cyclopentyl, cyclohexyl or furfuryl, or is a radical from the series phenyl and benzyl which is unsubstituted or is mono- to tri-substituted by fluorine, chlorine, bromine, methyl, methoxy or $CF_3$.

9. A pesticidal composition according to claim 8, characterised in that A is (p-phenoxy)phenyl; R is fluorine, chlorine, methyl, methoxy or $CF_3$; n is 0, 1, 2 or 3; $R_1$ is $OR_2$ or $SR_2$; wherein $R_2$ is $C_1$-$C_5$alkyl, allyl, propargyl, cyclohexyl or benzyl, or is phenyl which is unsubstituted or mono- to tri-substituted by fluorine, chlorine, bromine, methyl, methoxy or $CF_3$.

10. A pesticidal composition according to claim 1, characterised in that it is selected from the series:
[2,4-dichlorobenzyl-C-(tert.-butylthio)imino]pyrazine (no. 1.7);
[2,4-dichlorobenzyl-C-(isopropylthio)imino]pyrazine (no. 1.4).

11. A pesticidal composition according to any one of claims 1 to 10, characterised in that it contains from 0.1 to 99% of an active ingredient of formula I, from 99.9 to 1% of a solid or liquid adjuvant and from 0 to 25% of a surfactant.

12. A composition according to claim 11, characterised in that it contains from 0.1 to 95% of an active ingredient of formula I, from 99.8 to 5% of a solid or liquid adjuvant and from 0.1 to 25% of a surfactant.

13. A process for the preparation of compounds of formula I, characterised in that a compound of formula II

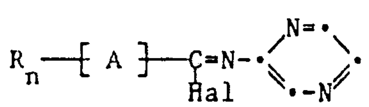
(II)

is reacted in the presence of a base with a compound of formula III

$$R_1\text{-}H \quad (III),$$

$R_n$, A and $R_1$ in formulae II and III being as defined for formula I, and Hal being halogen, preferably chlorine or bromine.

14. A process for the preparation of an agrochemical composition, characterised in that at least one compound of formula I according to any one of claims 1 to 12 is homogeneously mixed with suitable solid or liquid adjuvants and surfactants.

15. A method of controlling or preventing an attack on cultivated plants by phytopathogenic microorganisms, characterised in that a compound of formula I is applied to the plant or to the locus thereof.

16. The use of compounds of formula I for controlling plant diseases that are attributable to microorganisms.

17. The use according to claim 16 of compounds of formula I according to any one of claims 3 to 10.

18. The use according to claim 16, characterised in that the microorganims are phytopathogenic fungi.

19. The use according to claim 18 against fungi from the classes Ascomycetes, Basidiomycetes or Fungi imperfecti.

**Revendications pour les Etats contractants:**
DE, GB, FR, CH, LI, IT, NL, BE, SE, LU

1. Composés de formule I

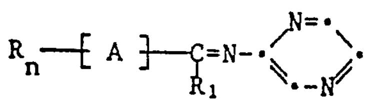

(I)

dans laquelle

A est le phényle, naphtyle, biphényle, phénoxyphényle ou phénylthiophényle;

R est un halogène, cyano, nitro, alkyle $C_1$-$C_4$, alcoxy $C_1$-$C_4$, haloalcoxy $C_1$-$C_4$, haloalkyle $C_1$-$C_4$ ou di(alkyl $C_1$-$C_4$)amino;

n est 0, 1, 2, 3, 4 ou 5;

$R_1$ représente l'un des groupes $OR_2$, $SR_2$ ou $N(R_3)(R_4)$, $R_2$ représentant un alkyle $C_1$-$C_8$, haloalkyle $C_1$-$C_4$, alcényle $C_3$-$C_8$, alcynyle $C_3$-$C_8$ ou un reste, non substitué ou substitué de une à trois fois par R, de la série phényle, benzyle, cycloalkyle $C_3$-$C_7$ ou furfuryle; et

$R_3$ et $R_4$ représentent indépendamment l'un de l'autre, l'hydrogène ou un alkyle $C_1$-$C_4$ ou constituent ensemble avec l'azote de l'amine un hétérocycle saturé à cinq ou six membres, qui comprend comme hétéroatome soit seulement l'azote de l'amine, soit comme autre hétéroatome N, O ou S.

2. Composés de formule I selon la revendication 1, caractérisés en ce que

A est le phényle, naphtyle, biphényle, phénoxyphényle ou phénylthiophényle;

R est un halogène, cyano, nitro, alkyle $C_1$-$C_4$, alcoxy $C_1$-$C_4$, haloalcoxy $C_1$-$C_4$, haloalkyle $C_1$-$C_4$ ou di(alkyl $C_1$-$C_4$)amino;

n est 0, 1, 2, 3, 4 ou 5;

$R_1$ représente l'un des groupes $OR_2$, $SR_2$ ou $N(R_3)(R_4)$, $R_2$ représentant un alkyle $C_1$-$C_8$, alcényle $C_3$-$C_8$, alcynyle $C_3$-$C_8$ ou un haloalkyle $C_1$-$C_4$, un reste, non substitué ou substitué de une à trois fois par R, de la série phényle, benzyle, cycloalkyle $C_3$-$C_7$ ou furfuryle; et

$R_3$ et $R_4$ représentent indépendamment l'un de l'autre, l'hydrogène ou un alkyle $C_1$-$C_4$ ou constituent ensemble avec l'azote de l'amine un hétérocycle saturé à cinq ou six membres, qui comprend comme hétéroatome, soit seulement l'azote de l'amine, soit comme autre hétéroatome N, O ou S.

3. Composés de formule I selon la revendication 1, caractérisés en ce que A est le phényle; R est halogène, cyano, méthyle, éthyle, méthoxy, éthoxy, $CF_3$, haloalcoxy $C_1$-$C_2$, $N(CH_3)_2$ ou $N(C_2H_5)_2$; n est 1, 2 ou 3; $R_1$ représente l'un des groupes $OR_2$ ou $SR_2$; $R_2$ représentant un alkyle $C_1$-$C_8$, alcényle $C_3$-$C_8$, alcynyle $C_3$-$C_8$ ou un reste, non substitué ou substitué de une à trois fois par un halogène, cyano, méthyle, éthyle, méthoxy, éthoxy, $CF_3$, haloalcoxy $C_1$-$C_2$ ou N(alkyl $C_1$-$C_2$)$_2$ de la série phényle, benzyle, cycloalkyle $C_5$-$C_6$ et furfuryle.

4. Composés de formule I selon la revendication 3, caractérisés en ce que A représente le phényle; R est le fluor, chlore, brome, méthyle, méthoxy ou $CF_3$; n est 1 ou 2; $R_1$ représente $OR_2$ ou $SR_2$; $R_2$ est un alkyle $C_1$-$C_5$, alcényle $C_3$-$C_4$, alcynyle $C_3$-$C_4$, cyclopentyle, cyclohexyle, furfuryle ou un reste, non substitué ou substitué de une à trois fois par le fluor, le chlore, le brome, méthyle, méthoxy ou $CF_3$, de la série phényle ou benzyle.

5. Composés de formule I selon la revendication 4, caractérisés en ce que R représente le fluor ou le chlore; n représente la position 2 et 4 du cycle phényle; $R_1$ représente $OR_2$ ou $SR_2$; $R_2$ étant un alkyle $C_1$-$C_5$, allyle, propargyle, cyclohexyle, benzyle ou un phényle non substitué ou substitué de une à trois fois par le fluor, chlore, brome, méthyle, méthoxy ou $CF_3$.

6. Composés de formule I selon la revendication 1, caractérisés en ce que, A est le phénoxyphényle; R est halogène, cyano, méthyle, éthyle, méthoxy, éthoxy, $CF_3$, haloalcoxy $C_1$-$C_2$, $N(CH_3)_2$ ou $N(C_2H_5)_2$; n est 0, 1, 2,3, 4 ou 5; $R_1$ représente l'un des groupes $OR_2$ ou $SR_2$; $R_2$ représentant un alkyle $C_1$-$C_8$, alcényle $C_3$-$C_8$, alcynyle $C_3$-$C_8$ ou un reste, non substitué ou substitué de une à trois fois par un halogène, cyano, méthyle, éthyle, méthoxy, éthoxy, $CF_3$, haloalcoxy $C_1$-$C_2$ ou N(alkyl $C_1$-$C_2$)$_2$, de la série phényle, benzyle, cycloalkyle $C_5$-$C_6$ ou furfuryle.

7. Composés de formule I selon la revendication 6, caractérisés en ce que A est le (p-phénoxy)-phényle.

8. Composés de formule I selon la revendication 7, caractérisés en ce que, A est le (p-phénoxy)phényle; R est le fluor, chlore, brome, méthyle, méthoxy ou $CF_3$; n est 0, 1, 2, 3 ou 4; $R_1$ représente $OR_2$ ou $SR_2$; $R_2$ est un alkyle $C_1$-$C_5$, alcényle $C_3$-$C_4$, alcynyle $C_3$-$C_4$, cyclopentyle, cyclohexyle, furfuryle ou un reste, non substitué ou substitué de une à trois fois par le fluor, le chlore, le brome, méthyle, méthoxy ou $CF_3$, de la série phényle ou benzyle.

9. Composés de formule I selon la revendication 8, caractérisés en ce que, A est le (p-phénoxy)-phényle; R est le fluor, chlore, brome, méthyle, méthoxy ou $CF_3$; n est 0, 1, 2 ou 3; $R_2$ est un alkyle $C_1$-$C_5$, allyle, propargyle, cyclohexyle, benzyle ou un phényle non substitué ou substitué de une à trois fois par le fluor, le chlore, le brome, méthyle, méthoxy ou $CF_3$.

10. Composé de formule I selon la revendication 1, caractérisé en ce qu'il est choisi dans la série:

[2,4-dichlorobenzyl-C-(tert.butylthio)imino]pyrazine (n° 1.7);

[2,4-dichlorobenzyl-C-(isopropylthio)imino]pyrazine (n° 1.4).

11. Procédé de préparation de composés de formule I, caractérisé en ce qu'on fair réagir un composé de formule II

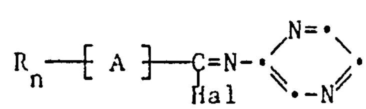

(II)

en présence d'une base avec un composé de formule III

$R_1$-H (III)

dans ce cas, $R_n$, A et $R_1$ dans les formules II et III ont les significations données pour la formule I, Hal représente un halogène, de préférence le chlore ou le brome.

12. Moyen de lutte contre les parasites pour combattre ou empêcher une attaque par des microorganismes, caractérisé en ce qu'il comprend comme composant actif au moins un composé de formule I selon la revendication 1.

13. Moyen selon la revendication 12, caractérisé en ce qu'il comprend comme composant actif au moins un composé de formule I selon la revendication 3.

14. Moyen pour combattre des microorganismes selon la revendication 12, caractérisé en ce qu'il comprend au moins comme composant actif un composé de formule I selon la revendication 4.

15. Moyen pour combattre des microorganismes selon la revendication 12, caractérisé en ce qu'il comprend comme composant actif au moins un composé de formule I selon l'une des revendications 5 à 10.

16. Moyen selon l'une des revendications 12 à 15, caractérisé en ce qu'il comprend de 0,1 à 99% d'un principe actif de formule I, 99,9 à 1% d'un additif solide ou liquide et de 0 à 25% d'un tensioactif.

17. Moyen selon la revendication 16, caractérisé en ce qu'il comprend de 0,1 à 95% d'un principe actif de formule I, 99,8% à 5% d'un additif solide ou liquide et 0,1 à 25% d'un tensioactif.

18. Procédé de préparation d'un moyen agrochimique, caractérisé en ce qu'on mélange intimement un composé de formule I selon des revendications 12 à 17 avec des additifs appropriés solides ou liquides et des tensioactifs.

19. Procédé pour combattre ou empêcher une attaque de plantes cultivées par des microorganismes phytopathogènes, caractérisé en ce qu'on applique un composé de formule I sur les plantes ou l'endroit où elles poussent.

20. Utilisation de composés de formule I pour combattre des maladies de plantes qui sont provoquées par des microorganismes.

21. Utilisation selon la revendication 20 de composés de formule I selon l'une des revendications 3 à 10.

22. Utilisation selon la revendication 20, caractérisée ne ce que les microorganismes sont des champignons pathogènes.

23. Utilisation selon la revendication 22 contre des champignons de la classe ascomycètes, basidiomycètes ou fungi imperfecti.

24. Composé de formule II

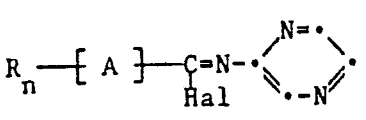

(II)

dans laquelle $R_n$ et A ont les significations données pour la formule I et Hal représente un halogène, de préférence le chlore ou le brome.

25. Composé de formule VI

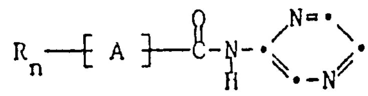

(VI)

dans laquelle $R_n$ et A ont les significations données pour la formule I.

**Revendications pour l'Etat contractant:** AT

1. Moyen de lutte contre les parasites pour combattre ou empêcher l'attaque par des microorganismes, caractérisé en ce qu'il contient au moins comme composant actif un composé de formule I

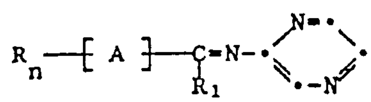

(I)

dans laquelle

A est le phényle, naphtyle, biphényle, phénoxyphényle ou phénylthiophényle;

R est un halogène, cyano, nitro, alkyle $C_1$-$C_4$, alcoxy $C_1$-$C_4$, haloalcoxy $C_1$-$C_4$, haloalkyle $C_1$-$C_4$ ou di(alkyl $C_1$-$C_4$)amino;

n est 0, 1, 2, 3, 4 ou 5;

$R_1$ représente l'un des groupes $OR_2$, $SR_2$ ou $N(R_3)(R_4)$; $R_2$ représentant un alkyle $C_1$-$C_8$, haloalkyle $C_1$-$C_4$, alcényle $C_3$-$C_8$, alcynyle $C_3$-$C_8$ ou un reste, non substitué ou substitué de une à trois fois par R, de la série phényle, benzyle, cycloalkyle $C_3$-$C_7$ ou furfuryle; et

$R_3$ et $R_4$ représentent indépendamment l'un de l'autre, l'hydrogène ou un alkyle $C_1$-$C_4$ ou constituent ensemble avec l'azote de l'amine un hétérocycle saturé à cinq ou six membres, qui comprend comme hétéroatome soit seulement l'azote de l'amine, soit comme autre hétéroatome N, O ou S.

2. Moyen de lutte contre les parasites, caractérisés en ce que

A est le phényle, naphtyle, biphényle, phénoxyphényle ou phénylthiophényle;

R est un halogène, cyano, nitro, alkyle $C_1$-$C_4$, alcoxy $C_1$-$C_4$, haloalcoxy $C_1$-$C_4$, haloalkyle $C_1$-$C_4$ ou di(alkyl $C_1$-$C_4$)amino;

n est 0, 1, 2, 3, 4 ou 5;

$R_1$ représente l'un des groupes $OR_2$, $SR_2$ ou $N(R_3)(R_4)$, $R_2$ représentant un alkyle $C_1$-$C_8$, alcényle $C_3$-$C_8$, alcynyle $C_3$-$C_8$ ou haloalkyle $C_1$-$C_4$, ou un reste, non substitué ou substitué de une à trois fois par R, de la série phényle, benzyle, cycloalkyle $C_3$-$C_7$ ou furfuryle; et

$R_3$ et $R_4$ représentent indépendamment l'un de l'autre, l'hydrogène ou un alkyle $C_1$-$C_4$ ou constituent ensemble avec l'azote de l'amine un hétérocycle saturé à cinq ou six membres, qui comprend

comme hétéroatome, soit seulement l'azote de l'amine, soit comme autre hétéroatome N, O ou S.

3. Moyen de lutte contre les parasites selon la revendication 1, caractérisé en ce que A est le phényle; R est halogène, cyano, méthyle, éthyle, méthoxy, éthoxy, $CF_3$, haloalcoxy $C_1$-$C_2$, $N(CH_3)_2$ ou $N(C_2H_5)_2$; n est 1, 2 ou 3; $R_1$ représente l'un des groupes $OR_2$ ou $SR_2$; $R_2$ représentant un alkyle $C_1$-$C_8$, alcényle $C_3$-$C_8$, alcynyle $C_3$-$C_8$ ou un reste, non substitué ou substitué de une à trois fois par un halogène, cyano, méthyle, éthyle, méthoxy, éthoxy, $CF_3$, haloalcoxy $C_1$-$C_2$ ou N(alkyl $C_1$-$C_2$)$_2$ de la série phényle, benzyle, cycloalkyle $C_5$-$C_6$ et furfuryle.

4. Moyen de lutte contre les parasites selon la revendication 3, caractérisé en ce que A représente le phényle; R est le fluor, chlore, brome, méthyle, méthoxy ou $CF_3$; n est 1 ou 2; $R_1$ représente $OR_2$ ou $SR_2$; $R_2$ est un alkyle $C_1$-$C_5$, alcényle $C_3$-$C_4$, alcynyle $C_3$-$C_4$, cyclopentyle, cyclohexyle, furfuryle ou un reste, non substitué ou substitué de une à trois fois par le fluor, le chlore, le brome, méthyle, méthoxy ou $CF_3$, de la série phényle ou benzyle.

5. Moyen de lutte contre les parasites selon la revendication 4, caractérisé en ce que R représente le fluor ou le chlore; n représente la position 2 et 4 du cycle phényle; $R_1$ représente $OR_2$ ou $SR_2$; $R_2$ étant un alkyle $C_1$-$C_5$, allyle, propargyle, cyclohexyle, benzyle ou un phényle non substitué ou substitué de une à trois fois par le fluor, chlore, brome, méthyle, méthoxy ou $CF_3$.

6. Moyen de lutte contre les parasites selon la revendication 1, caractérisé en ce que, A est le phénoxyphényle; R est halogène, cyano, méthyle, éthyle, méthoxy, éthoxy, $CF_3$, haloalcoxy $C_1$-$C_2$, $N(CH_3)_2$ ou $N(C_2H_5)_2$; n est 0, 1, 2, 3, 4 ou 5; $R_1$ représente l'un des groupes $OR_2$ ou $SR_2$; $R_2$ représentant un alkyle $C_1$-$C_8$, alcényle $C_3$-$C_8$, alcynyle $C_3$-$C_8$ ou un reste, non substitué ou substitué de une à trois fois par un halogène, cyano, méthyle, éthyle, méthoxy, éthoxy, $CF_3$, haloalcoxy $C_1$-$C_2$ ou N(alkyl $C_1$-$C_2$)$_2$, de la série phényle, benzyle, cycloalkyle $C_5$-$C_6$ ou furfuryle.

7. Moyen de lutte contre les parasites selon la revendication 6, caractérisé en ce que A est le (p-phénoxy)-phényle.

8. Moyen de lutte contre les parasites selon la revendication 7, caractérisé en ce que A est le (p-phénoxy)phényle; R est le fluor, chlore, brome, méthyle, méthoxy ou $CF_3$; n est 0, 1, 2, 3 ou 4; $R_1$ représente $OR_2$ ou $SR_2$; $R_2$ est un alkyle $C_1$-$C_5$, alcényle $C_3$-$C_4$, alcynyle $C_3$-$C_4$, cyclopentyle, cyclohexyle, furfuryle ou un reste, non substitué ou substitué de une à trois fois par le fluor, le chlore, le brome, méthyle, méthoxy ou $CF_3$, de la série phényle ou benzyle.

9. Moyen de lutte contre les parasites selon la revendication 8, caractérisé en ce que A est le (p-phénoxy)phényle; R est le fluor, chlore, brome, méthyle, méthoxy ou $CF_3$; n est 0, 1, 2 ou 3; $R_1$ représente $OR_2$ ou $SR_2$; $R_2$ est un alkyle $C_1$-$C_5$, allyle, propargyle, cyclohexyle, benzyle ou un phényle non substitué ou substitué de une à trois fois par le fluor, le chlore, le brome, méthyle, méthoxy ou $CF_3$.

10. Moyen de lutte contre les parasites selon la revendication 1, caractérisé en ce qu'il est choisi dans la série:

[2,4-dichlorobenzyl-C-(tert.butylthio)imino]pyrazine (n° 1.7);

[2,4-dichlorobenzyl-C-(isopropylthio)imino]pyrazine (n° 1.4).

11. Moyen de lutte contre les parasites selon l'une des revendications 1 à 10, caractérisé en ce qu'il comprend de 0,1 à 99% d'un principe actif de formule I, 99,9 à 1% d'un additif solide ou liquide et de 0 à 25% d'un tensioactif.

12. Moyen selon la revendication 11, caractérisé en ce qu'il comprend de 0,1 à 95% d'un principe actif de formule I, 99,8 à 5% d'un additif solide ou liquide et de 0,1 à 25% d'un tensioactif.

13. Procédé de préparation de composés de formule I, caractérisé en ce qu'on fait réagir un composé de formule II

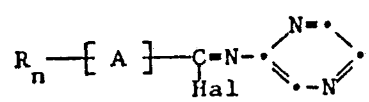

(II)

en présence d'une base avec un composé de formule III

$R_1$-H (III)

dans ce cas, $R_n$, A et $R_1$ dans les formules II et III ont les significations données pour la formule I, Hal représente un halogène, de préférence le chlore ou le brome.

14. Procédé de préparation d'un moyen agrochimique, caractérisé en ce qu'on mélange intimement un composé de formule I selon l'une des revendications 1 à 12 avec des additifs appropriés solides ou liquides et des tensioactifs.

15. Procédé pour combattre ou empêcher une attaque de plantes cultivées par des microorganismes phytopathogènes, caractérisé en ce qu'on applique un composé de formule I sur les plantes ou l'endroit où elles poussent.

16. Utilisation de composé de formule I pour combattre des maladies des plantes qui sont provoquées par des microorganismes.

17. Utilisation selon la revendication 16 de composés de formule I, selon l'une des revendications 3 à 10.

18. Utilisation selon la revendication 16, caractérisée en ce que les microorganismes sont des champignons pathogènes.

19. Utilisation selon la revendication 18 contre des champignons des classes ascomycètes, basidiomycètes ou fungi imperfecti.